# EUROPÄISCHE PATENTSCHRIFT

(11) **EP 0 959 092 B1**
(45) Veröffentlichungstag und Bekanntmachung des Hinweises auf die Patenterteilung: **18.08.2004**
(21) Anmeldenummer: 99109056.4
(22) Anmeldetag: 07.05.1999
(51) Int. Cl.: C08G 73/10, C08G 73/02, C08G 69/10, A61K 7/06, A61K 7/48, C11D 3/37

(54) **Verfahren zur Herstellung von Polyaminosäureestern durch Veresterung von sauren Polyaminosäuren oder Umesterung von Polyaminosäureestern**
Process of production of polyaminoacidesters through esterification of polyaminoacids or of transesterification of polyaminoacidesters
Procédé de production d'esters d'acide polyamine par estérification d'acide polyamine ou par transestérification d'esters d'acide polyamine

(30) Priorität: 20.05.1998 DE 19822602
(43) Veröffentlichungstag der Anmeldung: 24.11.1999
(73) Patentinhaber: Goldschmidt AG, 45127 Essen (DE)
(72) Erfinder: Duetsch, Michael Dr., 45239 Essen (DE); Grüning, Burghard Dr., 45134 Essen (DE); Rau, Harald, 45139 Essen (DE); Simpelkamp, Jörg Dr., 45130 Essen (DE); Weitemeyer, Christian Dr., 45134 Essen (DE)

(56) Entgegenhaltungen:
- DE-A- 19 541 699
- DE-A- 19 545 678
- US-A- 4 888 398

## Beschreibung

Die Erfindung beschreibt ein Verfahren zur Herstellung von Polyaminosäureestern durch Veresterung von in der Seitenkette Carbonsäure- und/oder Carboxylatgruppen tragenden Polyaminosäuren - im folgenden saure Polyaminosäuren genannt - insbesondere von Polyasparaginsäure mit Alkoholen, wobei homogene Produkte entstehen und der Veresterungsgrad durch die beschriebenen Bedingungen gezielt eingestellt werden kann. Gegebenenfalls können saure Polyaminosäuren zum Einsatz kommen, deren Säuregruppen bereits zum Teil verestert sind und deren Alkoholkomponente der Estergruppen durch das erfindungsgemäße Verfahren ausgetauscht wird, im folgenden Umesterung genannt, gegebenenfalls unter gleichzeitiger Veresterung freier Carbonsäuregruppen einhergehend mit einer Erhöhung des Veresterungsgrades. Gegebenenfalls wird zur Beschleunigung der Ver- oder der Umesterung ein Katalysator mit sauren, lewissauren, basischen oder lewisbasischen Eigenschaften eingesetzt.

Polyasparaginsäure, welche als biologisch abbaubares Komplexierungsmittel und Cobuilder in waschmitteln in den Blickpunkt des Interesses getreten ist, läßt sich bekanntermaßen durch alkalische Hydrolyse aus der unmittelbaren Synthesevorstufe Polysuccinimid (PSI, Anhydropolyasparaginsäure), erhalten, welches beispielsweise durch thermische Kondensation der Asparaginsäure oder aus Ammoniak und Maleinsäure gewonnen werden kann.

Zu Polyaminosäuren mit tensidischen Eigenschaften kann man zum Beispiel gelangen, indem die freien Carbonsäuregruppen von sauren Polyaminosäuren in N-Alkylamide oder in Ester überführt werden. Die von verschiedenen Arbeitsgruppen beschriebene Umsetzung von Polysuccinimid mit Aminen führt zu Polyasparaginsäureamiden (Kovacs et al., J. Med. Chem. 1967, 10, 904-7; Neuse, Angew. Makromol. Chem. 1991, 192, 35-50). Neri et al. führen die Ringöffnung von PSI mit Ethanolamin durch und erhalten Hydroxyethylaspartamide (J. Med. Chem. 1973, 16, 893-897, Macromol. Synth. 1982, 8, 25-29). DE 37 00 128 A und EP 0 458 079 A beschreiben die nachfolgende Veresterung derartiger Hydroxyethylderivate mit Carbonsäurederivaten. Copolymere Polyasparaginsäureester sind, wie in DE 195 45 678 A beschrieben, durch Kondensation von Monoalkylestern der Malein- oder der Fumarsäure unter Ammoniakzugabe erhältlich. In DE 195 45 678 A ist weiterhin beschrieben, daß copolymere Polyasparaginsäureester durch Umsetzung von Polysuccinimid mit Alkoholen und einer sich gegebenenfalls anschließenden Hydrolyse zugänglich sind.

Je nach Veresterungsgrad und Hydrophobie der Alkoholkomponente zeichnen sich Polyasparaginsäureester neben ihrer ausgesprochenen Mildheit und biologischen Abbaubarkeit durch hervorragende Eigenschaften als Stabilisatoren für O/W- und W/O-Emulsionen, als schaumstabilisierendes und schaumverstärkendes Cotensid in Reinigungsmitteln, als Dispergiermittel unterschiedlicher Pigmente, als Komplexierungsmittel für Metallkationen aus.

Es hat sich die Aufgabe gestellt, ein Verfahren bereitzustellen Polyaminosäureester, insbesondere Polyasparaginsäureester, durch Umsetzung der entsprechenden Polyaminosäuren mit Alkoholen herzustellen, wobei homogene Produkte entstehen und der Veresterungsgrad durch die im Verfahren einzustellenden Bedingungen bestimmt werden kann.

Diese Aufgabe wird erfindungsgemäß gelöst mit einem Verfahren zur Herstellung von homogenen Polyaminosäureestern durch Umsetzung von Polyaminosäuren, die in den Seitenketten Carbonsäure- und/oder Carboxylatgruppen enthalten mit hydroxygruppenhaltigen Verbindungen durch teilweise oder vollständige Veresterung oder durch Umesterung von teilveresterten Polyaminosäuren mit Alkoholen gegebenenfalls unter Erhöhung des Veresterungsgrades, unter Reaktionsbedingungen hoher Scherkräfte, gegebenenfalls unter Einsatz von Katalysatoren, Lösungsvermittlern und/oder vermindertem Druck.

Als saure Polyaminosäuren eignen sich beispielsweise Polyasparaginsäuren, Copolymerisate aus Asparaginsäure und wenigstens einer weiteren proteinogenen Aminosäure insbesondere Glutaminsäure, Hydrolysate von Polysuccinimid oder Umsetzungsprodukte von Maleinsäurederivaten, beispielsweise Maleinsäure, Maleinsäureanhydrid, Maleinsäureester und/oder Maleinsäureamide mit Ammoniak, gegebenenfalls in Gegenwart eine weiteren Aminosäure, insbesondere Glutaminsäure. Besonders bevorzugt ist der Einsatz von Polyasparaginsäure.

Die Polyaminosäuren können auch weitere Monomere enthalten, bevorzugt ist jedoch, daß im Mittel mindestens 30 Mol.-%, besonders bevorzugt, daß mindestens 60 Mol.-% der wiederkehrenden Einheiten in der Seitenkette eine Carbonsäure- und/oder eine Carboxylatgruppe aufweisen. Weitere Monomereinheiten können, gegebenenfalls in Form ihrer Derivate, beispielsweise Glutaminsäure, Glutamin, Asparagin, Lysin, Alanin, Glycin, Tyrosin, Tryptophan, Serin, Cystein, nicht proteinogene Aminosäuren mit jeweils einer oder mehreren Amino- bzw. Carboxyfunktionen, wie beispielsweise β-Alanin oder ω-Amino-1-alkansäuren, Di- oder Polycarbonsäuren wie beispielsweise Maleinsäure, Fumarsäure, Itaconsäure, Bernsteinsäure, Malonsäure, Adipinsäure, hydroxyfunktionelle Carbonsäuren, wie beispielsweise Weinsäure, Citronensäure, Apfelsäure oder deren Amide, Di- oder Polyhydroxyverbindungen, Di- oder Polyaminoverbindungen, Aminoalkohole mit einem linearen, verzweigten oder cyclischen, gesättigten, ungesättigten oder aromatischen Kohlenwasserstoffgerüst, gegebenenfalls oxo- oder aza-Analoge mit O- oder N-Atomen in der Kette, oder von Polyalkylenglykolen bzw. Ethylenoxid-Propylenoxid-Copolymeren umfassen.

Die jeweiligen Herstellverfahren der Ausgangspolyaminosäuren haben meist keinen Einfluß auf das erfindungsgemäße Verfahren.

In dem erfindungsgemäßen Verfahren können auch die entsprechenden Ammonium-, N-Alkylammonium, N,N-Dialkylammonium-, N,N,N-Trialkylammonium-, Alkali- oder Erdalkalisalze der beschriebenen Polyaminosäuren eingesetzt werden.

Es kann mit allen üblichen Verbindungen verestert oder umgeestert werden, die wenigstens eine Hydroxygruppe beinhalten. Dazu zählen Alkohole, einschließlich alkoxylierte Alkohole, Polyalkylenglykole, hydroxyhaltige Polymere, alkoxylierte Amine, Kohlenhydrate oder Hydroxycarbonsäuren einschließlich Zuckercarbonsäuren.

Insbesondere einwertige Alkohole im Sinne der vorliegenden Erfindung weisen 1 bis 24 C-Atome auf, beispielsweise Methanol, Ethanol, 1-Propanol, 2-Propanol, Allylalkohol, Propinol, 1-Butanol, 2-Butanol, 2-Methyl-2-propanol, 2-Methyl-1-butanol, 1-Pentanol, 2-Pentanol, 3-Methyl-1-butanol, Isopentanol (Isomerengemisch), 3-Methyl-3-butenol, 1-Hexanol, 2-Hexanol, 5-Hexen-1-ol, 3-Hexen-1-ol, Phenol, 1-Heptanol, Benzylalkohol, 1-Octanol, 2-Ethyl-1-hexanol, 1-Decanol, Citronellol, Linalool, 1-Dodecanol, 1-Tetradecanol, Farnesol, 1-Hexadecanol, 1-Octadecanol, Isostearylalkohol (Isomerengemisch), cis-9-Octadecen-1-ol oder 1-Eicosanol.

Beispiele für Alkohole mit mehreren Hydroxygruppen sind 1,2-Ethandiol, 1,2-Propandiol, 1,3-Propandiol, 1,4-Butandiol, 2,3-Butandiol, 2-Butin-1,4-diol, 1,6-Hexandiol, Trimethylolpropan, Pentaerythrit oder Zucker sowie Zuckerderivate.

Alkoxylierte Alkohole sind Umsetzungsprodukte von Alkoholen mit Epoxiden, beispielsweise mit Ethylenoxyd, Propylenoxyd, Butylenoxid oder Styroloxid oder Gemischen davon.

Hydroxyhaltige Polymere sind beispielsweise Polyethylenglykol, Polyoxyalkylene, insbesondere mit Monomereinheiten abgeleitet von Ethylenoxid, Propylenoxid, Butylenoxid oder Styroloxid, hydroxyalkylfunktionelle Acrylate und -methacrylate oder Polyvinylalkohol.

Es können auch Gemische der beschriebenen Alkohole eingesetzt werden.

Im Sinne der vorliegenden Erfindung werden vorzugsweise zwischen 0,1 und 5,0 Mol der hydroxygruppenhaltigen Verbindung je Mol freier Carbonsäure- und/oder Carboxylatgruppe eingesetzt. Für einen mittleren Veresterungsgrad von 20% im Endprodukt sind 0,2 bis 0,4 Mol hydroxygruppenhaltige Verbindung je Mol freier Carbonsäure- und/oder Carboxylatgruppe besonders bevorzugt. Für einen mittleren Veresterungsgrad von 35% im Endprodukt sind 0,35 bis 0,7 Mol hydroxygruppenhaltige Verbindung je Mol freier Carbonsäure- und/oder Carboxylatgruppe besonders bevorzugt. Für einen mittleren Veresterungsgrad von 50% im Endprodukt sind 0,5 bis 1,0 Mol hydroxygruppenhaltige Verbindung je Mol freier Carbonsäure- und/oder Carboxylatgruppe besonders bevorzugt. Für einen mittleren Veresterungsgrad von 75% im Endprodukt sind 0,75 bis 1,5 Mol hydroxygruppenhaltige Verbindung je Mol freier Carbonsäure- und/oder Carboxylatgruppe besonders bevorzugt.

Als saure Katalysatoren eignen sich alle Protonensäuren, beispielsweise Schwefelsäure, Natriumhydrogensulfat, Salzsäure, Phosphorsäure, Mononatriumdihydrogenphosphat, Dinatriumhydrogenphosphat, Pyrophosphorsäure, Phosphorige Säure, Unterphosphorige Säure, Salpetersäure, Methansulfonsäure, Trifluormethansulfonsäure, p-Toluolsulfonsäure, sulfonierte oder ionenaustauschende Harze.

Als Katalysatoren eignen sich weiterhin lewissaure bzw. lewisbasische oder basische Verbindungen, z. B. LiOH, NaOH, Na₂O, Na₂CO₃, NaHCO₃, NaF, NaH, NaNH₂, Na₂SO₄, Na₃PO₄, Natriumalkoholate, Natriumdodecylsulfat, Natriumtrifluoracetat, KOH, K₂O, K₂CO₃, KHCO₃, KCl, Kaliumalkoholate, KH, KNH₂, K₂SO₄, K₃PO₄, Kupferacetat, Cu(CO₃)₂, CuN(C₄H₉)₂, Cu(II)-Alkoholate, CuF₂, Kupfergluconat, Cu₃N, CuO, Ca(OH)₂, CaO, Calciumalkoholate, Ca(OOCCH₃)₂, CaCO₃, Ca(PO₄)₂, Mg(OH)₂, MgO, Magnesiumalkoholate, Mg(OOCCH₃)₂, MgCO₃, Mg(PO₄)₂ Zn(OOCCH₃)₂, Zn₃B₄O₉, ZnCl₂, Zn(OOCCH₂CH₃)₂, ZnF₂, Zinkgluconat, Zn(NO₃)₂, ZnC₂O₄, Alumina, AlCl₃, AlPO₄, Al(PO₃)₃, Al₂Si₂O₇, AlNiZn, Al₂O(OOCCH₃)₄, AlBr₃, Aluminiumalkoholate, Al(OC₃C₇)₂(C₆H₉O₃), Al(OH)₃, Aluminiumlactat, Al₂(MoO₄)₃, Al₂(SO₄)₃, Ti, TiO₂, Titannitrilotriacetat, Titaniumalkoholate, teilkondensierte Titaniumalkoholate, TiAl₃, TiH₂, TiNi, TiO, Ti₂O₃, Ti₂(SO₄)₃, ZrO₂, Zirconiumalkoholate, ZrAl₃, ZrH₂, ZrO, Zr(SO₄)₂, SiO₂, Zeolithe, Silicate, GeO₂, Sn(OOCCH₃), SnCl₂, Zinnalkoholate, PbO, PbO₂, VCl₃, VO, VO(C₅H₇O₂)₂, VOCl₃, tertiäre Amine, wie z. B. Trialkylamine insbesondere Triethylamin oder Triisopropylamin, 1,4-Diazabicyclo[2.2.2]octan, Sulfonamide, wie z. B. para-Toluolsulfonsäureamid, Triethanolamin, Pyridin, Alkylpyridine, Piperazin, Aminoxide, Ammoniumsalze, wie z. B. Tetramethylammoniumhydroxid, Tetrabutylammoniumchlorid, Tetrabutylammoniumbromid, P₂O₅, PCl₃, PCl₅, PBr₃, PBr₅, POCl₃, POBr₃, Sb, Sb₂O₅, SbCl₃, SbF₃, K(SbO) (C₄H₄O₆), Antimonalkoholate, Bi, BiCl₃, Bi(OOCCH₃)₃, Bismutalkoholate, MoCl₃, MoCl₄, MoCl₅, Molybdänalkoholate, MoO₂, MoO₃, W, WO₃*K₂O, WO₃*SiO₂, Wolframalkoholate, WCl₆, WO₂Cl₂, Sulfonsäurechloride, wie z. B. para-Toluolsulfonsäurechlorid, SCl₂, SO₂Cl₂, SO₃, MnCl₂, MnCO₃, Mn(OOCCH₃)₂, MnPO₄, Manganalkoholate, FeCl₃, FeCl₂, FeBr₃, FeF₃, Eisenalkoholate, Fe(NO₃)₃, Fe₃N, Eisenacetat, CoCl₂ CoCO₃, Kobaltalkoholate, Ni, Rh, Pd, Pt, PtCl₂, PtCl₄, K₂PtCl₆, CeCl₃, Ce₂(CO₃)₃, Ce(OOCCH₃)₃, (NH₄)₂Ce(NO₃)₆, Ce(OH)₄ und/oder Ceralkoholate.

Es können auch Gemische der beschriebenen Katalysatoren eingesetzt werden. Optional verbleibt der Katalysator im Endprodukt.

Die Katalysatoren werden in Mengen von 0,0001 Gew.-% bis 20 Gew.-%, bevorzugt 0,001 Gew.-% bis 10 Gew.-%, besonders bevorzugt 0,1 Gew.-% bis 5 Gew.-% bezogen auf die gesamte Reaktionsmischung eingesetzt.

Als bevorzugte Reaktoren zur Durchführung des erfindungsgemäßen Verfahren werden solche verwendet, in denen die Reaktionsmischung einer hohen Scherwirkung ausgesetzt ist, welche sowohl die Durchmischung der Reaktionskomponenten fördert, als auch die Destillation des während der Veresterung entstehenden Wassers und/oder des während einer Umesterung enstehenden Alkohols aus der viskosen Reaktionsmischung erleichtert. Dieses können beispielsweise Rührreaktoren mit scherkraftreichen Rührern, wie etwa Mig- oder Intermigrührer, Kaskaden verschiedener Rührreaktoren, ein- oder mehrwellige Extruder, Kneter, Knetreaktoren, ein- oder mehrwellige Hochviskosreaktoren, oder Schaufeltrockner sein. Besonders bevorzugt sind Reaktoren mit mehrstufigen Intermigrührern oder Hochviskosreaktoren, beispielsweise der Typen ORP, CRP oder DTB der LIST AG (Arisdorf, Schweiz).

Optional schließt sich das erfindungsgemäße Verfahren direkt an die Herstellung der Polyaminosäure an, wobei wahlweise der dazu eingesetzte Reaktor Verwendung finden kann oder das erfindungsgemäße Verfahren in einem weiteren, gegebenenfalls kaskadenartig angeschlossenen, Reaktor folgt.

Als Lösungsvermittler können handelsübliche Lösungsmittel eingesetzt werden, wie beispielsweise n-Alkane, Cycloalkane, Benzol, Toluol, Alkohole, beispielsweise Isopropanol, tert.-Butanol, sec.-Butanol, Isobutanol, Cyclopentanol, Cyclohexanol, Umsetzungsprodukte von Alkoholen mit Propylenoxid und/oder Ethylenoxid, Ketone, beispielsweise Aceton, Butanon, Methylisopropylketon, Methylisobutylketon, Ester, N,N-Dimethylformamid, N,N-Dimethylacetamid, Dimethylsulfoxyd oder N-Methylpyrrolidon.

Weitere Lösungsvermittler können hydrophob modifizierte Polyaminosäuren, insbesondere teilveresterte Polyasparaginsäuren, teilveresterte Polyasparaginsäure-co-Glutaminsäuren oder N-Alkylamide von Polyasparaginsäuren, beispielsweise hergestellt nach DE 195 45 678 A, sein. Das Herstellverfahren dieser Polyaminosäurederivate, insbesondere der teilveresterten Polyasparaginsäuren oder der teilveresterten Polyasparaginsäure-co-Glutaminsäuren hat praktisch keinen Einfluß auf deren lösungsvermittelnden Wirkung. Gegebenenfalls können diese Lösungsvermittler nach dem erfindungsgemäßen Verfahren hergestellt werden, in diesem speziellen Fall können 1 bis 80 Gew.-%, bevorzugt 10 bis 30 Gew.-% der Produktmischung im Reaktor verbleiben und als Lösungsvermittler für eine folgende Umsetzung dienen.

Weitere Lösungsvermittler können tensidische Verbindungen sein, die in der Lage sind, zumindest teilweise eine Emulsion bzw. eine Dispersion der Polyaminosäure oder des Polyaminosäurederivates in der hydroxygruppenhaltigen Verbindung zu stabilisieren. Dieses können anionische, amphotere, zwitterionische, kationische oder nichtionische Tenside sein, sowie Gemische daraus.

Anionische Tenside können beispielsweise aus der Gruppe der Sulfate, Sulfonate, Carboxylate sowie Mischungen derselben sein, beispielsweise Alkylbenzolsulfonate, α-Olefinsulfonate, α-sulfonierte Fettsäureester, Fettsäureglycerinestersulfate, Paraffinsulfonate, Alkylsulfate, Alkylpolyethersulfate, Sulfobernsteinsäurealkylester, Fettsäuresalze (Seifen), Fettsäureester der Polymilchsäure, N-Acylamino-säureester, N-Acyltaurate, Acylisethionate, Ethercarboxy-late, Monoalkylphosphate, N-Acylaminosäurederivate wie N-Acylaspartate oder N-Acylglutamate, N-Acylsarcosinate, Polyasparaginsäurederivate und andere.

Die anionischen Gruppen können in neutralisierter Form vorliegen, mit kationischen Gegenionen aus der Gruppe der Alkalimetalle, Erdalkalimetalle, Ammonium oder substituiertem Ammonium. Amphotere oder zwitterionische Tenside sind beispielsweise Alkylbetaine, Alkylamidoalkylbetaine des Typs Cocoamidopropylbetain, Sulfobetaine, Phosphobetaine, Sultaine und Amidosultaine, Imidazoliniumderivate, Amphoglycinate und andere.

Nichtionische Tenside sind beispielsweise oxethylierte Fettalkohole, oxethylierte Alkylphenole, oxethylierte Fettsäureester, oxethylierte Mono-, Di- oder Triglyceride oder Polyalkylenglykolfettsäureester. Andere nichtionische Tenside können aus der Gruppe der Alkylpolysaccharide, beispielsweise Alkyl- oder Alkenylpolyglucoside sowie deren Ethoxylierungsprodukte, Zukkerester, beispielsweise Fettsäureester der Glucose, Saccharose, Fructose oder des Methylglucosids, Sorbitolfettsäureester und Sorbitanfettsäureester (gegebenenfalls oxethyliert), Polyglycerinester, Fettsäurealkanolamide, N-Acylaminozuckerderivate, beispielsweise N-Acylglucamine, langkettige tertiäre Aminoxide oder Phosphinoxide sowie Dialkylsulfoxide stammen.

Kationische Tenside können aus der Gruppe der quarternären Ammoniumverbindungen, quarternisierte Proteinhydrolysate, Alkylamidoamine, quarternäre Esterverbindungen, quarternären Siliconöle, quarternäre Zuckerderivate sowie Mischungen derselben sein, beispielsweise Cetyltrimethylammoniumchlorid, Stearyltrimethylammoniumchlorid, Distearyldimethylammoniumchlorid, Tricetylmethylammoniumchlorid, Stearylamidopropyldimethylamin und Dialkylammoniummethosulfate.

Die in Kombination mit den erfindungsgemäßen Polyasparaginsäurederivaten eingesetzten Tenside können beliebige Kombinationen aus einem oder mehr Tensiden der obengenannten Kategorie sein.

Es können auch Gemische der beschriebenen Lösungsvermittler eingesetzt werden. Optional verbleibt der Lösungsvermittler im Endprodukt, insbesondere diejenigen Lösungsvermittler mit einem Siedepunkt von mehr als 200 °C.

Die Lösungsvermittler können in Mengen 0 bis 95 Gew.-%, bevorzugt 0 bis 50 Gew.-%, besonders bevorzugt 0 bis 25 Gew.-% bezogen auf die gesamte Reaktionsmischung eingesetzt werden.

Die Temperatur sollte während der Reaktion 0°C bis 250°C, bevorzugt 50°C bis 200°C, besonders bevorzugt 90°C bis 150°C betragen. Gegebenenfalls dient ein Temperaturgradient dem Fortschritt der Reaktion.

Der Druck sollte während der Reaktion Werte zwischen 5 bar und 0,0001 bar, besonders bevorzugt zwischen 1 bar und 0,001 bar erreichen.

Das bei der Veresterung entstehende Wasser wird vorzugsweise durch Destillation entfernt. Gegebenenfalls wird die Destillation durch verminderten Druck gefördert. Der Einsatz von Lösungsmitteln, die mit Wasser ein Azeotrop bilden, erleichtert ebenfalls den Fortschritt der Veresterung.

Werden teilveresterte Polyaminosäuren der Umesterung unterworfen, hat die austretende Alkoholkomponente bevorzugt einen niedrigeren Siedepunkt als die eintretende hydroxygruppenhaltige Verbindung und kann durch Destillation gegebenenfalls unter vermindertem Druck abdestilliert werden.

Je nach Veresterungsgrad und Hydrophobie der Alkoholkomponente können die nach dem erfindungsgemäßen Verfahren hergestellten Polyaminosäureester als Tenside, als Stabilisatoren für O/W- und W/O-Emulsionen, als schaumstabilisierende und schaumverstärkende Cotenside in Reinigungsmitteln, als Dispergiermittel unterschiedlicher Pigmente oder als Komplexierungsmittel für Metallkationen und/oder als Builder/Cobuilder in Waschmitteln eingesetzt werden.

Die nach dem erfindungsgemäßen Verfahren hergestellten Polyaminosäureester können als O/W-Emulgatoren für kosmetische Emulsionen eingesetzt werden, beispielsweise für Lotionen mit einer vergleichsweise niedrigen Viskosität oder Cremes und Salben mit einer hohen Viskosität, für Anwendungen als Hautpflegemittel wie beispielsweise Tagescremes, Nachtcremes, Pflegecremes, Nährcremes, Bodylotions, Salben und dergleichen. Als weitere Hilfs- und Zusatzstoffe können übliche Coemulgatoren, Konsistenzgeber, Ölkörper, Überfettungsmittel, Fette, Wachse, Stabilisatoren, Wirkstoffe, Glycerin, Farb- und Duftstoffe enthalten sein.

Als Konsistenzgeber können hydrophile Wachse, beispielsweise C₁₂-C₃₀-Fettalkohole, C₁₆-C₂₂-Fettsäuren, Glycerinmono- und - diester und Sorbitanmono- und diester von gesättigten Fettsäuren mit 12 bis 22 C-Atomen eingesetzt werden.

Als weitere Coemulgatoren kommen beispielsweise in Frage: Anlagerungsprodukte von 2 bis 30 Mol Ethylenoxid und /oder 0 bis 5 Mol Propylenoxid an C₁₂-C₃₀-Fettalkohole und Wollwachsalkohole, vorzugsweise lineare, gesättigte C₁₆-C₂₂-Fettalkohole; Ethylenoxidanlagerungsprodukte von Glycerinmono- und -diestern und Sorbitanmono- und diestern von gesättigten und ungesättigten Fettsäuren mit 6 bis 22 C-Atomen; Anlagerungsprodukte von 2 bis 30 Mol Ethylenoxid und /oder 0 bis 5 Mol Propylenoxid an Fettsäuren mit 12 bis 22 C-Atomen und an Alkylphenole mit 8 bis 15 C-Atomen in der Alkylgruppe; C₁₂-C₁₈-Fettsäurepartialester von Anlagerungsprodukten von 1 bis 30 Mol Ethylenoxid an Glycerin; Anlagerungsprodukte von Ethylenoxid an Fette und Öle, beispielsweise Rizinusöl oder gehärtetes Rizinusöl; Partialester von gesättigten oder ungesättigten C₁₂-C₂₂-Fettsäuren, auch verzweigte oder hydroxysubstituierte, mit Polyolen, beispielsweise Ester von Glycerin, Ethylenglykol, Polyalkylenglykolen, Pentaerythrit, Polyglycerin, Zuckeralkoholen wie Sorbit und Polyglucosiden wie Cellulose; Polysiloxan-Polyalkyl-Polyether-Copolymere und deren Derivate sowie hydrophob modifizierte Polyasparaginsäurederivate.

Als Coemulgatoren können auch anionische, kationische, nichtionische, amphotere und/oder zwitterionische Tenside beispielsweise aus der als verträglichkeitsfördernde Agenzien bezeichneten Gruppe sein.

Es können jeweils beliebige Mischungen der o.g. Konsistenzgeber und Coemulgatoren eingesetzt werden.

Als Ölkörper kommen beispielsweise Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₂₀-Fettsäuren mit verzweigten Alkoholen, Ester von linearen und/oder verzweigten C₆-C₂₀-Carbonsäuren mit mehrwertigen Alkoholen und/oder Guerbetalkoholen, Triglyceride auf Basis von C₆-C₁₀-Fettsäuren, pflanzliche und tierische Öle und Fette, verzweigte primäre Alkohole, substituierte Cyclohexane, Guerbetcarbonate, Dialkylether und/oder aliphatische bzw. naphthenische Kohlenwasserstoffe in Betracht.

Als Überfettungsmittel können beispielsweise Lanolin und Lecithinderivate sowie deren Ethoxylate, Polyolfettsäureester, Monoglyceride und Fettsäurealkanolamide verwendet werden. Es können Siliconverbindungen wie Polydimethylsiloxane, Cyclodimethicone sowie amino-, fettsäure-, alkohol-, epoxy-, fluor, und/oder alkylmodifizierte Siliconverbindungen sowie Wachse wie beispielsweise Bienenwachs, Paraffinwachse oder Mikrowachse enthalten sein. Die Emulsionen können Verdickungsmittel wie Polyacrylsäurederivate oder kationische Polymere wie z.B. kationische Cellulose- oder Stärkederivate, kationische Chitin oder Chitosanderivate, kationische Siliconpolymere, Copolymere von Diallylammoniumsalzen z.B. mit Acrylamiden, Polyethylenimin enthalten. Weiterhin können Metallsalze von Fettsäuren, z.B. Magnesium-, Aluminium- oder Zinkstearat als Stabilisatoren oder Zinksalze der Ricinolsäure als Geruchshemmer enthalten sein. Es können übliche Sonnenschutzwirkstoffe wie Titandioxid, p-Aminobenzoesäure etc., Duftstoffe, Farbstoffe, biogene Wirkstoffe wie Pflanzenextrakte oder Vitaminkomplexe sowie pharmazeutische Wirkstoffe enthalten sein. Weiterhin können die Emulsionen Perlglanzmittel wie Ethylenglykoldistearat sowie die üblichen Konservierungsmittel wie Parabene, Sorbinsäure, Phenoxyethanol und andere enthalten.

Die erfindungsgemäßen Polyasparaginsäurederivate können auch in W/O-Emulsionen eingesetzt werden, beispielsweise als Emulgatoren und/oder Coemulgatoren für die Herstellung von Hautpflegecremes und -lotionen.

Die erfindungsgemäßen Polyasparaginsäurederivate mit einem naturnahen Polyaminosäurerückgrat sind milde Tenside, welche alleine oder in Kombination mit anionischen, kationischen, nichtionischen, zwitterionischen und/oder amphoteren Tensiden eingesetzt werden können. Es sind feste, flüssige oder pastöse Zubereitungen möglich, z.B. Seifenstücke, Waschlotionen, Duschgele, Shampoos.

Die in Kombination mit den nach dem erfindungsgemäßen Verfahren hergestellten Polyaminosäureestern einsetzbaren Tenside können anionische, kationische, amphotere oder zwitterionische oder nichtionische Tenside sein, beispielsweise aus den schon als Lösungsvermittler genannten Verbindungsklassen.

Die erfindungsgemäßen Tensidzubereitungen können weitere Hilfs- und Zusatzstoffe enthalten wie beispielsweise Wasser und Lösungsmittel z.B. aus der Gruppe der Alkohole und Polyole, Verdickungsmittel, Trübungsmittel, z.B. Glykolesterderivate; Moisturizer, Emollients wie tierische und pflanzliche Öle, Carbonsäureester, Lanolin, Bienenwachs, Silicone; polymere Agenzien zur Verbesserung des Hautgefühls, konditionierende, pflegende oder pharmazeutisch wirksame Bestandteile wie z.B. kationische oder amphotere Polymere, Proteine und Proteinderivate, Lanolinderivate, Panthothensäure, Betain, Polydimethylsiloxane oder deren Derivate, Sonnenschutzwirkstoffe sowie Lösungsvermittler, Stabilisatoren, Geruchsstoffe, Puffersubstanzen, Konservierungsmittel und/oder Farbstoffe.

Tensidzubereitungen, die die nach dem erfindungsgemäßen Verfahren hergestellten Polyaminosäureester enthalten, lassen sich vorteilhaft anwenden in z.B. Haarshampoos, Duschbäder, Schaumbadzubereitungen, Hand-, Gesichts und Intimreinigungslotionen, Flüssigseifen, Seifenstücke, Rasiercremes, Handwaschpasten, hautfreundlichen Geschirrspülmitteln, Reinigungsmitteln für glatte Oberflächen sowie in Zahncremes.

Die nach dem erfindungsgemäßen Verfahren hergestellten Polyaminosäureester können als Dispergiermittel beispielsweise für Lacke und Farben eingesetzt werden. Dort bewirken sie eine günstige Farbstärkeentwicklung sowie eine Verbesserung der Rub-Out-Eigenschaften.

Die nach dem erfindungsgemäßen Verfahren hergestellten Polyaminosäureester werden dazu vorteilhaft mit dem Stand der Technik entsprechenden Neutralisationsmitteln, insbesondere Aminen neutralisiert. Insbesondere bevorzugt ist hier die Verwendung von Dimethylethanolamin oder 2-Amino-2-methylpropanol. Zur Herstellung wäßriger Pigmentpasten werden 0,1-100 Gew.-%, vorzugsweise 0,5-50 Gew.-%, insbesondere 2 bis 15 Gew.-% bezogen auf das Gewicht der Pigmente verwendet. Die nach dem erfindungsgemäßen Verfahren hergestellten Polyaminosäureester können bei der erfindungsgemäßen Verwendung entweder vorab mit den zu dispergierenden Pigmenten vermischt werden oder direkt in dem Dispergiermedium (Wasser, eventuelle Glycolzusätze) vor oder gleichzeitig mit der Zugabe der Pigmente und etwaiger anderer Feststoffe gelöst werden. Die Neutralisation kann dabei vor oder während der Herstellung der Pigmentpasten erfolgen. Bevorzugt werden nach dem erfindungsgemäßen Verfahren hergestellte Polyaminosäureester eingesetzt, deren freie Carbonsäuregruppen bereits partiell oder vollständig neutralisiert wurden.

Die nach dem erfindungsgemäßen Verfahren hergestellten Polyaminosäureester können auch in beliebigen Gemischen mit weiteren, dem Stand der Technik entsprechenden Dispergieradditiven, beispielsweise aus der Gruppe der Fettsäurealkoxylate, Poly(meth)acrylate, Polyester, Polyether etc., eingesetzt werden.

Als Pigmente können in diesem Zusammenhang beispielsweise anorganische oder organische Pigmente, sowie Ruße genannt werden. Füllstoffe, die beispielsweise in wäßrigen Lacken dispergiert werden können, sind beispielsweise solche auf Basis von Kaolin, Talkum, anderen Silikaten, Kreide, Glasfasern, Glasperlen oder Metallpulvern. Als anorganische Pigmente seien exemplarisch genannt Titandioxid und Eisenoxide. In Betracht zu ziehende organische Pigmente sind beispielsweise Azopigmente, Metallkomplex-Pigmente, Phthalcyaninpigmente, anthrachinoide Pigmente, polycyclische Pigmente, insbesondere solche der Thioindigo-, Chinacridon-, Dioxazin-, Pyrrolopyrrol-, Naphthalintetracarbonsäure-, Perylen-, Isoamidolin(on)-, Flavanthron-, Pyranthron- oder Isoviolanthron-Reihe.

Als Lacksysteme, in denen die erfindungsgemäßen Pigmentpasten aufgelackt werden können, kommen beliebige wäßrige 1K- oder 2K-Lacke in Betracht. Beispielhaft genannt seien wäßrige 1K-Lacke wie beispielsweise solche auf Basis von Alkyd-, Acrylat-, Epoxid-, Polyvinylacetat-, Polyester- oder Polyurethanharzen oder wäßrige 2K-Lacke, beispielsweise solchen auf Basis von hydroxylgruppenhaltigen Polyacrylat- oder Polyesterharzen mit Melaminharzen oder gegebenenfalls blockierten Polyisocyanatharzen als Vernetzer. In gleicher Weise seien auch Polyepoxidharzsysteme genannt.

Die nach dem erfindungsgemäßen Verfahren hergestellten Polyaminosäureester können als komplexbildende Agenzien z.B. in Waschmitteln, als Inkrustationsinhibitoren, als Metalldesaktivatoren in Kunststoffen, als Hilfsstoffe in der Leder- und Textilindustrie oder als wirkungsverstärkende Zusatzstoffe zu Pestiziden oder Insektiziden eingesetzt werden. Hochmolekulare Derivate eignen sich auch als Absorbermaterialien.

### Beispiele:

### Vergleichsbeispiel 1

In einem 100 ml Vierhalsrundkolben, bestückt mit einem Flügelrührer, einem Innenthermometer und einer Destillationsbrücke, wurden 11,5 g Polyasparaginsäure (M_{w} = 6000 n. GPC, Eichung gegen Polyacrylat) mit 9,3 g (0,05 Mol, entspr. 0,5 Äquivalente je Asparaginsäureeinheit) n-Dodecanol und 1 g konz. Schwefelsäure versetzt und auf 150°C erhitzt. Diese Temperatur wurde unter Rühren und Normaldruck während 8 h beibehalten. Nach Abkühlen erhielt man ein inhomogenes Produkt (Gemisch Polyasparaginsäure, Fettalkohol, Polyasparaginsäureester), laut ¹³C-NMR-Spektrum waren weniger als 10% der Asparaginsäuregruppen verestert.

### Vergleichsbeispiel 2

In einem 100 ml Vierhalsrundkolben, bestückt mit einem Propellerrührer, einem Innenthermometer und einer Destillationsbrücke, wurden 11,5 g Polyasparaginsäure (M_{w} = 6000 n. GPC, Eichung gegen Polyacrylat) mit 48,4 g (0,2 Mol, entspr. 2,0 Äquivalente je Asparaginsäureeinheit) n-Hexadecanol, 3 g Phosphorsäure und 10 ml Dimethylformamid versetzt und auf 150°C erhitzt. Diese Temperatur wurde unter Rühren und einem auf 100 mbar reduzierten Druck während 8 h beibehalten. Nach Abkühlen erhielt man ein inhomogenes Produkt (Gemisch Polyasparaginsäure, Fettalkohol, Polyasparaginsäureester), laut ¹³C-NMR-Spektrum waren weniger als 10% der Asparaginsäuregruppen verestert.

### Vergleichsbeispiel 3

In einem 100 ml Vierhalsrundkolben, bestückt mit einem Propellerrührer, einem Innenthermometer und einer Destillationsbrücke, wurden 5,75 g Polyasparaginsäure (M_{w} = 6000 n. GPC, Eichung gegen Polyacrylat) mit 18,6 g (0,1 Mol, entspr. 1,0 Äquivalent je Asparaginsäureeinheit) n-Dodecanol und 1,7 g Methansulfonsäure versetzt und auf 150°C erhitzt. Diese Temperatur wurde unter Rühren und einem auf 100 mbar reduzierten Druck während 4 h beibehalten. Nach Abkühlen erhielt man ein inhomogenes Produkt (Gemisch Polyasparaginsäure, Fettalkohol, Polyasparaginsäureester), laut ¹³C-NMR-Spektrum waren weniger als 10% der Asparaginsäuregruppen verestert.

### Beispiel 1

In einem 2 l Planschliffkolben, bestückt mit einem zweistufigen Intermigrührer, einem Innenthermometer und einer Destillationsbrücke, wurden 575 g Polyasparaginsäure (M_{w} = 6000 n. GPC, Eichung gegen Polyacrylat) mit 807 g (3,3 Mol, entspr. 0,67 Äquivalente je Asparaginsäureeinheit) n-Dodecanol, 60 g Methansulfonsäure und 140 ml Dimethylformamid versetzt und auf 140°C erhitzt. Diese Temperatur wurde unter Rühren und einem auf 100 mbar reduzierten Druck während 4 h beibehalten. Nach Abkühlen erhielt man ein homogenes Produkt, laut ¹³C-NMR-Spektrum waren 40% der Asparaginsäuregruppen verestert.

### Beispiel 2

Zum Einsatz kam ein Hochviskosreaktor CRP 2.5 Batch der Firma LIST AG mit zwei horizontalliegenden, gleichlaufenden, beheizten und selbstreinigenden Rührwellen und innenliegender Austragsschnecke, der Antrieb erfolgte mit einem Hydraulikaggregat. Der Brüdenausgang wurde mit einem Rückflußkühler mit Wasserabscheider versehen. In dieser Apparatur wurden 1295 g Polyasparaginsäure (M_{w}= 1800 n. GPC, Eichung gegen Polyacrylat) mit 415 g (5,6 Mol, entspr. 0,5 Äquivalente je Asparaginsäureeinheit) n-Butanol und 85 g Methansulfonsäure versetzt, anschl. wurde das Wärmeträgeröl im Heizmantel des Hochviskosreaktors auf 140°C erhitzt. Die Rührgeschwindigkeit wurde auf 35/44 U/min eingestellt. Nach 5 h nahm die Wassermenge im Wasserabscheider nicht mehr zu. Der Rückflußkühler wurde gegen eine Destillationsbrücke ausgetauscht und unter Druckverminderung auf 25 mbar wurde das restliche Butanol abdestilliert. Nach Abkühlen erhielt man ein homogenes Produkt, laut ¹³C-NMR-Spektrum waren 34% der Asparaginsäuregruppen verestert.

### Beispiel 3

Zum Einsatz kam ein Hochviskosreaktor CRP 2.5 Batch der LIST AG mit zwei horizontalliegenden, gleichlaufenden, beheizten und selbstreinigenden Rührwellen und innenliegender Austragsschnecke, der Antrieb erfolgte mit einem Hydraulikaggregat. Der Brüdenausgang wurde mit einer Destillationsbrücke versehen. In dieser Apparatur wurden 805 g Polyasparaginsäure (M_{w} = 1800 n. GPC, Eichung gegen Polyacrylat) mit 1016 g (4,2 Mol, entspr. 0,6 Äquivalente je Asparaginsäureeinheit) n-Hexadecanol, 100 ml Dimethylformamid und 96 g p-Toluolsulfonsäure versetzt, anschl. wurde das Wärmeträgeröl im Heizmantel des Hochviskosreaktors auf 150°C erhitzt und der Druck auf 25 mbar vermindert. Die Rührgeschwindigkeit wurde auf 35/44 U/min eingestellt. Nach 6,5 h wurde die Umsetzung beendet. Nach Abkühlen erhielt man ein homogenes Produkt, laut ¹³C-NMR-Spektrum waren 42% der Asparaginsäuregruppen verestert.

### Beispiel 4

Zum Einsatz kam ein Hochviskosreaktor CRP 2.5 Batch der LIST AG mit zwei horizontalliegenden, gleichlaufenden, beheizten und selbstreinigenden Rührwellen und innenliegender Austragsschnecke, der Antrieb erfolgte mit einem Hydraulikaggregat. Der Brüdenausgang wurde mit einer Destillationsbrücke versehen. In dieser Apparatur wurden 320 g Dodecylpolyaspartat (Veresterungsgrad: 20 %), 1150 g Polyasparaginsäure (M_{w} = 1800 n. GPC, Eichung gegen Polyacrylat) mit 460 g (2,5 Mol, entspr. 0,25 Äquivalente je Asparaginsäureeinheit) n-Dodecanol und 80 g Methansulfonsäure versetzt, anschl. wurde das Wärmeträgeröl im Heizmantel des Hochviskosreaktors auf 140°C erhitzt und der Druck auf 25 mbar vermindert. Die Rührgeschwindigkeit wurde auf 35/44 U/min eingestellt. Nach 6,5 h wurde die Umsetzung beendet. Anschließend wurden 85 Gew.-% der Reaktionsmischung über die Austragsschnecke abgenommen, welches zu einem homogenen Produkt erstarrte und laut ¹³C-NMR-Spektrum einen Veresterungsgrad von 21% bezogen auf die Asparaginsäuregruppen aufwies. Zu der im Reaktor bei erhöhter Temperatur (Wärmeträgeröl: 140°C) verbliebenen Reaktionsmischung wurden 1150 g Polyasparaginsäure (M_{w} = 1800 n. GPC, Eichung gegen Polyacrylat), 460 g (2,5 Mol, entspr. 0,25 Äquivalente je Asparaginsäureeinheit) n-Dodecanol und 68 g Methansulfonsäure gegeben und der Druck auf 25 mbar vermindert. Nach 6 h wurde die Umsetzung beendet. Man erhielt ein homogenes Produkt, laut ¹³C-NMR-Spektrum waren 19% der Asparaginsäuregruppen verestert.

### Beispiel 5

Zum Einsatz kam ein Hochviskosreaktor CRP 2.5 Batch der LIST AG mit zwei horizontalliegenden, gleichlaufenden, beheizten und selbstreinigenden Rührwellen und innenliegender Austragsschnecke, der Antrieb erfolgte mit einem Hydraulikaggregat. Der Brüdenausgang wurde mit einem Tropftrichter versehen. In dieser Apparatur wurden 823 g (8,4 Mol) Maleinsäureanhydrid, 551,3 g (2,6 Mol) Glutaminsäure und 280 g Wasser vorgelegt, die Rührgeschwindigkeit wurde auf 35/44 U/min eingestellt, anschl. wurde das Wärmeträgeröl im Heizmantel des Hochviskosreaktors auf 100°C erhitzt und, 582 g (8,6 Mol) 25%ige wäßrige Ammoniak-Lösung während 10 min durch den Tropftrichter zugegeben. Während der Ammoniakdosierung wurde die Temperatur des Wärmeträgeröls auf 120°C erhöht. Unter langsamer Druckverminderung auf 7 mbar wurde die Temperatur des Wärmeträgeröls auf 150°C erhöht. Nach insgesamt 7 h endete die Destillation, es wurde auf 120°C abgekühlt. Im Anschluß wurden 799 g (3,3 Mol) n-Hexadecanol und 70 g Methansulfonsäure zu der ausdestillierten Reaktionsmischung gegeben und das Wärmeträgeröl für 6 h unter Verminderung des Drucks auf 25 mbar auf 140 °C erhitzt. Nach Abkühlen erhielt man ein homogenes Produkt, laut ¹³C-NMR-Spektrum waren 27% der Asparaginsäuregruppen verestert.

### Beispiel 6

Zum Einsatz kam ein Hochviskosreaktor CRP 2.5 Batch der LIST AG mit zwei horizontalliegenden, gleichlaufenden, beheizten und selbstreinigenden Rührwellen und innenliegender Austragsschnecke, der Antrieb erfolgte mit einem Hydraulikaggregat. Der Brüdenausgang wurde mit einer Destillationsbrücke versehen. In dieser Apparatur wurden 900 g Butylpolyaspartat (Veresterungsgrad: 34 %) des Beispiels 2 mit 968 g (4,0 Mol, entspr. 1,75 Äquivalente je Asparaginsäurebutylestereinheit) n-Hexadecanol und 100 g Titantetraisopropylat versetzt, anschl. wurde das Wärmeträgeröl im Heizmantel des Hochviskosreaktors auf 140°C erhitzt. Die Rührgeschwindigkeit wurde auf 35/44 U/min eingestellt. Nach 6,5 h wurde die Umsetzung beendet. Man erhielt ein homogenes Produkt, laut ¹³C-NMR-Spektrum waren 37% der Asparaginsäuregruppen mit Hexadecyl- und 8% der Asparaginsäuregruppen mit Butylresten verestert.

Die Beispiele zeigen eindeutig, daß mit dem erfindungsgemäßen Verfahren homogene Produkte erhalten werden, die mit Verfahren nach dem Stands der Technik (Vergleichsbeispiele 1-3) nicht zugänglich sind.

## Patentansprüche

1. Verfahren zur Herstellung von homogenen Polyaminosäureestern durch Umsetzung von Polyaminosäuren, die in den Seitenketten Carbonsäure- und/oder Carboxylatgruppen enthalten mit hydroxygruppenhaltigen Verbindungen durch teilweise oder vollständige Veresterung oder Umesterung unter Reaktionsbedingungen hoher Scherkräfte, gegebenenfalls unter Einsatz von Katalysatoren, Lösungsvermittlern und/oder vermindertem Druck.

2. Verfahren nach Anspruch 1, **dadurch gekennzeichnet, daß** als Polyaminosäure Polyasparaginsäure oder Polyasparaginsäure-co-glutaminsäure eingesetzt wird.

3. Verfahren nach Anspruch 1 oder 2, **dadurch gekennzeichnet, daß** man Carbonsäureestergruppen der eingesetzten Polyaminosäure, gegebenenfalls unter gleichzeitiger Veresterung von Carbonsäure- und/oder Carboxylatgruppen, umestert.

4. Verfahren nach einem der Ansprüche 1 bis 3, **dadurch gekennzeichnet, daß** die hydroxygruppenhaltige Verbindung einen oder mehrere Alkohole mit 1 bis 24 C-Atomen, gegebenenfalls alkoxyliert, umfaßt.

5. Verfahren nach einem der Ansprüche 1 bis 4, **dadurch gekennzeichnet, daß** die Katalysatoren saure, basische, lewissaure oder lewisbasische Verbindungen oder deren Gemische umfassen.

6. Verfahren nach einem der Ansprüche 1 bis 5, **dadurch gekennzeichnet, daß** das Verfahren bei einer Temperatur von 0 bis 250 °C sowie einem Druck von 0,0001 bis 5 bar durchgeführt wird.

7. Verfahren nach einem der Ansprüche 1 bis 6, **dadurch gekennzeichnet, daß** während der Reaktion das entstehende Wasser und/oder andere niedrigsiedende Reaktionsprodukte abdestilliert werden.

8. Verfahren nach Anspruch 1 bis 7, **dadurch gekennzeichnet, daß** ein Rührkessel mit mehrstufigen Mig- oder Intermigrührern oder ein Hochviskosreaktor mit mindestens einer horizontalliegenden Rührwelle, die gegebenenfalls beheizt werden kann, verwendet wird.

9. Verfahren nach einem der Ansprüche 1 bis 8, **dadurch gekennzeichnet, daß** der Reaktionsmischung ein Lösungsvermittler in einer Menge von 0 bis 95 Gew.-%, bezogen auf die Reaktionsmischung, insbesondere 0 bis 40 Gew.-%, bezogen auf die Reaktionsmischung, zugesetzt wird.

10. Verfahren nach Anspruch 9, **dadurch gekennzeichnet, daß** als Lösungsvermittler Lösungsmittel mit einem Siedepunkt von weniger als 200 °C bei Normaldruck, welches vorzugsweise mit Wasser ein Azeotrop bildet, und/oder teilveresterte Polyasparaginsäure und/oder teilveresterte Polyasparaginsäure-co-glutaminsäure und/oder tensidische Verbindungen aus der Gruppe der anionischen, kationischen, nichtionischen, amphoteren oder zwitterionischen Tenside eingesetzt werden.

11. Verfahren nach einem der Ansprüche 1 bis 10, **dadurch gekennzeichnet, daß** nach der Veresterung 1 bis 40 Gew.-%, insbesondere 15 bis 30 Gew.-%, des Produkts als Lösungsvermittler für Folgereaktionen im Reaktor verbleibt, wobei die Teilung der abreagierten Reaktionsmischung in Produkt und Lösungsvermittler für eine Folgereaktion beliebig häufig wiederholt werden kann.

12. Verfahren nach einem der Ansprüche 1 bis 11, **dadurch gekennzeichnet, daß** man Asparaginsäure- und/oder Maleinsäurederivate gegebenenfalls in Gegenwart von proteinogenen Aminosäuren, insbesondere Glutaminsäuren, gegebenenfalls in Gegenwart von Ammoniak in situ umsetzt und das Umsetzungsprodukt als Polyaminosäure einsetzt.

13. Verwendung der nach einem der Ansprüche 1 bis 12 erhältlichen Produkte in kosmetischen W/O- oder O/W-Emulsionen.

14. Verwendung der Emulsionen nach Anspruch 13 als Hautpflegemittel, Tagescremes, Nachtcremes, Pflegecremes, Nährcremes, Bodylotions, pharmazeutische Salben und Lotionen, Aftershavelotionen sowie Sonnenschutzmittel.

15. Verwendung der nach einem der Ansprüche 1 bis 12 erhältlichen Produkte in tensidischen Zubereitungen für Reinigungsmittel und/oder kosmetische Mittel, gegebenenfalls enthaltend ein oder mehrere weitere Tenside aus der Gruppe der anionischen, kationischen, nichtionischen, amphoteren und zwitterionischen Tenside sowie deren Mischungen daraus neben üblichen Hilfs- und Zusatzstoffen.

16. Verwendung der Tensidzubereitungen nach Anspruch 15 für Shampoos, Waschlotionen und Reinigungsmittel für Gesicht, Haar, Haut und Intimbereich, Rasiercreme und -lotionen, Flüssigseifen, Geschirrspülmittel, Reinigungsmittel für glatte Oberflächen, Seifenstücke, Schaumbäder, Duschgele sowie in Zahncremes und/oder Mundspülungen.

17. Verwendung der nach einem der Ansprüche 1 bis 12 erhältlichen Produkte als Waschmittelhilfsstoff, Komplexagens für mehrwertige Kationen, Dispergierhilfsmittel für Lacke und Farben, Inkrustationsinhibitor, Builder oder Co-builder für Waschmittel, Absorbermaterialien, Metalldes-aktivatoren in Kunststoffen, als Hilfsstoffe in der Leder und Textilindustrie oder als wirkungsverstärkende Zusatzstoffe zu Pestiziden oder Insektiziden.

## Claims

1. Process for the preparation of homogeneous polyamino acid esters by reaction of polyamino acids which contain carboxylic acid and/or carboxylate groups in the side chains with compounds which contain hydroxyl groups by partial or complete esterification or transesterification under reaction conditions of high shear forces, with or without the use of catalysts, solubilizers and/or reduced pressure.

2. Process according to Claim 1, **characterized in that** the polyamino acid is polyaspartic acid or a polyaspartic acid-co-glutamic acid.

3. The process according to Claim 1 or 2, **characterized in that** carboxylic ester groups of the polyamino acid used are transesterified optionally with simultaneous esterification of carboxylic acid and/or carboxylate groups.

4. The process according to one of Claims 1 to 3, **characterized in that** the compound containing hydroxyl groups includes one or more alcohols having from 1 to 24 carbon atoms, optionally alkoxylated.

5. The process according to one of Claims 1 to 4, **characterized in that** the catalysts are acidic, basic, Lewis acid or Lewis base compounds or mixtures thereof.

6. The process according to one of Claims 1 to 5, **characterized in that** the process is carried out at a temperature of from 0 to 250°C and a pressure of from 0.0001 to 5 bar.

7. The process according to one of Claims 1 to 6, **characterized in that**, during the reaction, the water formed and/or other low-boiling reaction products are distilled off.

8. The process according to Claims 1 to 7, **characterized in that** a stirred vessel with multistage mig or intermig stirrers or a high-viscosity reactor with at least one horizontal stirrer shaft, which can optionally be heated, is used.

9. The process according to one of Claims 1 to 8, **characterized in that** a solubilizer is added to the reaction mixture in an amount of from 0 to 95% by weight, based on the reaction mixture, in particular from 0 to 40% by weight, based on the reaction mixture.

10. The process according to Claim 9, **characterized in that** the solubilizer is a solvent with a boiling point of less than 200°C at atmospheric pressure, which preferably forms an azeotrope with water, and/or partially esterified polyaspartic acid and/or partially esterified polyaspartic acid-co-glutamic acid and/or surface-active compounds from the group of anionic, cationic, nonionic, amphoteric or zwitterionic surfactants.

11. The process according to one of Claims 1 to 10, **characterized in that**, following esterification, from 1 to 40% by weight, in particular from 15 to 30% by weight, of the product remains in the reactor as solubilizer for subsequent reactions, it being possible to repeat the separation of the fully reacted reaction mixture into product and solubilizer for a subsequent reaction as often as desired.

12. The process according to one of Claims 1 to 11, **characterized in that** aspartic acid derivatives and/or maleic acid derivatives are reacted in situ, optionally in the presence of proteinogenic amino acids, in particular glutamic acids, optionally in the presence of ammonia, and the reaction product is used as polyamino acid.

13. Use of the products obtainable according to one of Claims 1 to 12 in cosmetic W/O or O/W emulsions.

14. Use of the emulsions according to Claim 13 as skin care compositions, day creams, night creams, care creams, nourishing creams, body lotions, pharmaceutical ointments and lotions, aftershave lotions and sunscreens.

15. Use of the products obtainable according to one of Claims 1 to 12 in surface-active preparations for cleaners and/or cosmetic compositions, optionally comprising one or more further surfactants from the group of anionic, cationic, nonionic, amphoteric and zwitterionic surfactants and mixtures thereof together with customary auxiliaries and additives.

16. Use of surface-active preparations according to Claim 15 for shampoos, washing lotions and cleansers for face, hair, skin and intimate areas, shaving creams and lotions, liquid soaps, dishwashing detergents, cleaners for smooth surfaces, soap bars, bubble baths, shower gels and in toothpastes and/or mouthwashes.

17. Use of the products obtainable according to one of Claims 1 to 12 as detergent auxiliary, complexing agent for polyvalent cations, dispersion auxiliary for coatings and colourants, encrustation inhibitor, builder or cobuilder for detergents, absorber materials, metal deactivators in plastics, as auxiliaries in the leather and textile industry or as activity-enhancing additives for pesticides or insecticides.

## Revendications

1. Procédé pour la préparation de poly(esters d'acide aminé) homogènes par transformation de poly (acides aminés) qui contiennent dans les chaînes latérales des groupes acide carboxylique et/ou carboxylate avec des composés contenant des groupes hydroxy par estérification ou transestérification partielle ou complète dans des conditions de réaction avec des forces de cisaillement élevées, le cas échéant avec utilisation de catalyseurs, de promoteurs de solubilisation et/ou d'une pression réduite.

2. Procédé selon la revendication 1, **caractérisé en ce qu'**on utilise comme poly(acide aminé) le poly(acide aspartique) ou le poly(acide aspartique-co-acide glutamique).

3. Procédé selon la revendication 1 ou 2, **caractérisé en ce qu'**on transestérifie des groupes ester d'acide carboxylique des poly(acides aminés) utilisés, le cas échéant avec estérification simultanée des groupes acide carboxylique et/ou carboxylate.

4. Procédé selon l'une quelconque des revendications 1 à 3, **caractérisé en ce que** le composé contenant des groupes hydroxy comprend un ou plusieurs alcools comprenant 1 à 24 atomes de carbone, le cas échéant alcoxylés.

5. Procédé selon l'une quelconque des revendications 1 à 4, **caractérisé en ce que** les catalyseurs comprennent des composés acides, basiques, acides de Lewis ou bases de Lewis ou leurs mélanges.

6. Procédé selon l'une quelconque des revendications 1 à 5, **caractérisé en ce que** le procédé est réalisé à une température de 0 à 250°C ainsi qu'à une pression de 0,0001 à 5 bars.

7. Procédé selon l'une quelconque des revendications 1 à 6, **caractérisé en ce qu'**on élimine par distillation l'eau et/ou les autres produits de réaction à bas point d'ébullition formés pendant la réaction.

8. Procédé selon les revendications 1 à 7, **caractérisé en ce qu'**on utilise une cuve agitée avec des agitateurs MIG à plusieurs étages ou Intermig ou un réacteur à haute viscosité avec au moins un arbre d'agitation en position horizontale qui peut le cas échéant être chauffé.

9. Procédé selon l'une quelconque des revendications 1 à 8, **caractérisé en ce qu'**on ajoute au mélange réactionnel un promoteur de solubilisation en une quantité de 0 à 95% en poids par rapport au mélange réactionnel, en particulier de 0 à 40% en poids par rapport au mélange réactionnel.

10. Procédé selon la revendication 9, **caractérisé en ce qu'**on utilise comme promoteur de solubilisation des solvants présentant un point d'ébullition inférieur à 200°C à pression normale qui forme de préférence un azéotrope avec l'eau et/ou du poly(acide aspartique) partiellement estérifié et/ou du poly(acide aspartique-co-acide glutamique) partiellement estérifié et/ou des composés tensioactifs du groupe des agents tensioactifs anioniques, cationiques, non ioniques, amphotères ou zwittérioniques.

11. Procédé selon l'une quelconque des revendications 1 à 10, **caractérisé en ce qu'**après l'estérification, 1 à 40% en poids, en particulier 15 à 30% en poids, du produit reste dans le réacteur comme promoteur de solubilisation pour les réactions ultérieures, la division du mélange réactionnel qui a réagi en produit et promoteur de solubilisation pour une réaction ultérieure pouvant être répétée aussi souvent que voulu.

12. Procédé selon l'une quelconque des revendications 1 à 11, **caractérisé en ce qu'**on transforme in situ des dérivés de l'acide aspartique et/ou de l'acide maléique, le cas échéant en présence d'acides aminés protéinogènes, en particulier les acides glutamiques, le cas échéant en présence d'ammoniaque et on utilise le produit de transformation comme poly(acide aminé).

13. Utilisation des produits pouvant être obtenus selon l'une quelconque des revendications 1 à 12 dans des émulsions cosmétiques E/H ou H/E.

14. Utilisation des émulsions selon la revendication 13 comme agents de soin de la peau, crèmes de jour, crèmes de nuit, crèmes de soin, crèmes nourrissantes, lotions pour le corps, pommades et lotions pharmaceutiques, lotions après-rasage ainsi qu'agents de protection contre le soleil.

15. Utilisation des produits pouvant être obtenus selon l'une quelconque des revendications 1 à 12 dans des compositions tensioactives pour des produits de nettoyage et/ou des agents cosmétiques, contenant le cas échéant un ou plusieurs autres agents tensioactifs du groupe des agents tensioactifs anioniques, cationiques, non ioniques, amphotères et zwittérioniques ainsi que les mélanges de ceux-ci, outre les adjuvants et additifs usuels.

16. Utilisation des compositions tensioactives selon la revendication 15 pour des shampooings, des lotions de lavage et de nettoyage pour le visage, les cheveux, la peau et les zones intimes, des crèmes de rasage et des lotions de rasage, des savons liquides, des produits de vaisselle, des agents de nettoyage pour surfaces lisses, des briques de savon, des bains moussants, des gels de douche ainsi que dans les dentifrices et/ou les produits de rinçage pour la bouche.

17. Utilisation des produits pouvant être obtenus selon l'une quelconque des revendications 1 à 12 comme adjuvants de lavage, complexants pour des cations polyvalents, adjuvants de dispersion pour des laques et des peintures, inhibiteur d'incrustations, adjuvants ou coadjuvants pour agents de lavage, matériaux absorbants, désactivateurs métalliques dans les matériaux synthétiques, adjuvants dans le cuir et l'industrie du textile ou comme additifs renforçant l'activité dans les pesticides et les insecticides.
